Europäisches Patentamt

(19) European Patent Office

Office européen des brevets

(11) Veröffentlichungsnummer: **0 147 498**

**A2**

(12) # EUROPÄISCHE PATENTANMELDUNG

(21) Anmeldenummer: **84101961.5**

(22) Anmeldetag: **24.02.84**

(51) Int. Cl.⁴: **G 01 N 33/48**

(30) Priorität: **28.07.83 DE 3327248**

(43) Veröffentlichungstag der Anmeldung:
**10.07.85 Patentblatt 85/28**

(84) Benannte Vertragsstaaten:
**AT BE CH DE FR GB IT LI NL SE**

(71) Anmelder: **Institut Dr. Ziegler
Wyhlenweg 18
CH-4126 Bettingen(CH)**

(72) Erfinder: **Thorn, Werner, Prof. Dr.
Flemingstrasse 11
D-2000 Hamburg 60(DE)**

(72) Erfinder: **Ziegler, Walter Johann, Dr.
Wyhlenweg 18
CH-4126 Bettingen/Basel(CH)**

(74) Vertreter: **Kressin, Horst-Rüdiger, Dr.
DIEHL & KRESSIN Flüggenstrasse 17
D-8000 München 19(DE)**

(54) **Verfahren zum Testen von chemischen und biologischen Substanzen.**

(57) Die Erfindung betrifft ein Verfahren zum Testen von Substanzen auf ihre zellschädigende Wirkung, wobei Gewebe mit den zu untersuchenden Schadsubstanzen oder einem Gemisch aus Schadsubstanz und Natriumpyruvat unter Kontrolle der Bildung von Plasmapolypen behandelt wurde und der Gehalt von Plasmapolypen nach der Einwirkung in dem behandelten, exzidierten Gewebe durch Stichproben in histologischen Schnitten qualitativ und an der gesamten Magenschleimhaut quantitativ bestimmt wird.

EP 0 147 498 A2

Croydon Printing Company Ltd.

0147498

Verfahren zum Testen von
chemischen und biologischen Substanzen

Beschreibung

Die Erfindung betrifft ein Testverfahren für chemische
und biologische Substanzen, die für Arzneimittel, Lebensmittel, Insektizide, Pestizide, Lösungsmittel und andere
Güter des täglichen Bedarfs vorgesehen oder in ihnen enthalten sind im Hinblick auf ihre zellschädigende Wirkung,
wobei tierisches oder menschliches Gewebe mit Substanzen,
gegebenenfalls zusammen mit Natriumpyruvat, unter Bildung
von Plasmapolypen (zellorganellarme Protrusionen), welche
als Maß für die zellschädigende Wirkung zu gelten haben,
behandelt wurde und der Gehalt an Plasmapolypen nach der
Einwirkung der Substanzen in dem behandelten exzidierten
Gewebe in histologischen Schnitten bestimmt wird.

Testverfahren sind im allgemeinen Mittel, mit welchen die
Funktion menschlicher und tierischer Organe überwacht und
Veränderungen aufgespürt werden. Dabei handelt es sich um
chemisch/biochemische Analysen, fortlaufende Registrierungen
und histologische Kontrollen.

Eine besondere Gruppe stellen Analyseverfahren dar, mit deren
Hilfe Art und Menge von Arzneimitteln, Genuß- und Suchtmitteln sowie Umweltgiften ermittelt werden können, z.B. die
Bestimmung des Blutalkoholspiegels, des Herzglycosidspiegels
usw. Bezüglich der zu untersuchenden biologischen Flüssigkeiten stehen das Blut, Blutplasma und Urin im Vordergrund,
ferner der Stuhl, Drüsensekrete, wie Speichel, Magensaft,
Galle, Pankreasaft, Schweiß, Rückenmarksflüssigkeit sowie
Exsudate in Brust, Bauch und Gelenken. Untersuchungen an
Organen orientieren sich an der Fragestellung.

Im Hinblick auf die vielfältigen Anforderungen an ein Diagnostikum bezüglich Krankheitsverlaufskontrolle, Therapiekontrolle und Therapiesteuerung sowie an deren hohen Bedarf wird die wiederholte Anwendung zunächst auf einfache, preisgünstige Verfahren beschränkt, um dann im Laufe der Zeit immer mehr auf spezifische Diagnostika für die Kontrolluntersuchungen überzugehen.

Stets auf der Suche nach bequemer zu handhabenden und auch spezielleren Verfahren durch den Übergang von Einmal- zu Mehrfachuntersuchungen am gleichen Objekt und aufgrund der Nicht-Toxizität einer Substanzkategorie (in vitro-Diagnose) und nicht zuletzt die Vorsorge der Bevölkerung aus gesundheitspolitischen Gründen sowie das Vorsorgebedürfnis des einzelnen Menschen nimmt die Bedeutung der Kontrollverfahren zu.

Eine besondere Rolle spielen unter den Kontrollverfahren die Funktionsdiagnostika zur Beurteilung der physiologischen Funktion von Einzelorganen oder Organsystemen. Diesen Diagnostika verwandt sind solche, die die Sekretion endokriner Drüsen zu stimulieren vermögen und daher ein Maß für die Funktionstüchtigkeit liefern, insbesondere für den Magen, das Pankreas usw.

Es wurden bereits Testverfahren zur Funktionskontrolle für die Beurteilung der Schädlichkeit einer Substanz hinsichtlich ihrer zellschädigenden Wirkung am Beispiel einer Plasmapolypenbildung studiert.

In Res. exp. Med. 171 (1977) Seiten 155 bis 162 werden beispielsweise bereits Versuche zur in-vitro-Erzeugung von Plasmapolypen (PP) in menschlichen Placenten post partum beschrieben, um die PP in einem Arbeitsgang durch die umgekehrte Zentrifugation zu konzentrieren und möglichst rein zu gewinnen.

Im Archiv für Gynäkologie, 244 (1977) Seite 195 werden ferner Untersuchungen über die Placentagängigkeit von Acetylsalicylsäure nach parenteralen Gaben an die Mutter unter der Geburt und in Fortschr. med. 89 (1971) Seiten 1159 bis 1162 über die Nebenwirkungen von Salicylaten auf den menschlichen Organismus berichtet.

Auch ist es bereits bekannt, daß die Vergiftung trächtiger Meerschweinchen mit Monojodacetat oder Natriumfluorid als Hemmer des Kohlenhydratabbaus innerhalb weniger Minuten zu signifikanten morphologischen Veränderungen am Syncytiotrophoblasten der Placenta führt. Neben Schwellungen der Mitochondrien, des endoplasmatischen Reticulums und des Golgiapparates sowie einer Kernpyknose treten in großer Zahl Plasmapolypen auf (0,5 bis 20 µm große, organellarme Protrusionen). In Langzeitversuchen kontrolliert bis zu 10 Tagen, führen solche PP-Nester zu Placentainfarkten. Auch die histologischen Kontrollen an Humanplacenten, die zwecks Inkubation im Schüttelbad in schmale Streifen aufgeschnitten worden waren, ergaben eine verstärkte PP-Bildung aus dem Syncytiotrophoblasten.

Die vermehrte Plasmapolypenbildung als Zeichen für eine Schwangerschaftsstörung ist aus "Archiv für Gynäkologie", 216 (1974), Seite 175 bis 176 bekannt. In tierexperimentellen Untersuchungen zur systemischen Erforschung der Ursachen der PP-Bildung wurde durch Gaben von Natriumfluorid (NaF) oder Monojodacetat (MJA) eine verstärkte, reproduzierbare Bildung und Abschnürung von Zellprotrusionen in Meerschweinchenplacenten nach Blockade des Glucoseabbaus erreicht. Hingegen führten Vergiftungen mit 2.4-Dinitrophenol zu keiner vermehrten PP-Bildung (vgl. Archiv für Gynäkologie, 221 (1976), Seite 203).

Die Schwierigkeit in der Beurteilung der Ergebnisse bekannter Bestimmungsmethoden liegt vor allem darin begründet, daß bei vielen Substanzen, die auf einen menschlichen oder tierischen Organismus einwirken, eine Schädigung erst nach mehrfacher Anwendung oder langer Kontaktzeit (Weichmacher in Kunststoffen, halogenierte Produkte etc.) eintritt, die Schadstoffwirkung am Anfang unbemerkt bleibt und dann nur durch aufwendige Untersuchungen erfaßbar wird. Nachdem ein einfacher, schneller Nachweis im histologischen Präparat bisher nicht in jedem Fall möglich war, war man darauf angewiesen, systematische Versuchsreihen an Labortieren bei hohem Kosten- und Zeitaufwand durchzuführen.

Die Abschnürung von Zellprotrusionen auf Schadstoffeinwirkungen und mechanische Läsionen ist allen tierischen und menschlichen Geweben, unabhängig vom Geschlecht, eigen (in Zellkulturen getestet an Blutstammzellen, Hepatocyten, Fibroblasten etc.).

Dieser Befund gilt z.B. auch für die kernlosen Erythrocyten und nicht nur für den Synzytiotrophoblasten. In dem Synzytiotrophoblasten, der für den Austausch der Blutgase jedweder Substrate und der Stoffwechselendprodukte zwischen Mutter und Foeten in seiner Gesamtheit verantwortlich ist, werden die Zellmembranen sehr rasch abgebaut. Aus diesem Grund kommen für die quantitative Erfassung der Zellprotrusionen die meisten Gewebe nicht in Frage, weil man die Plasmapolypen (PP) nicht quantitativ aus dem Gewebeverband gewinnen kann. So bedarf es zur quantitativen Erfassung der PP relativ größer, zugänglicher Gewebeoberflächen, wie sie etwa bei der Placenta vorhanden sind (vgl. DE-PS 28 10 425). Allerdings gelang auch diese bekannte Methode der Erfassung an der Placenta nur nach völliger Blutfreispülung der Placenta und durch Bestimmung der Plasmapolypen mittels umgekehrter Zentrifugation, was mit erheblichem Zeitaufwand und der Zurverfügungstellung von reifen Tier- oder Humanplacenten verbunden war. Dies gilt auch für die Möglichkeit, Schadstoffeinwirkungen an Einzelzotten aus Humanplacenten speziell im Hinblick auf die Bildungsdauer und Größe der Plasmapolypen zu verfolgen.

Der vorliegenden Erfindung liegt die Aufgabe zugrunde, ein Testverfahren zu schaffen, mit dem bestimmte Schadwirkungen oder unerwünschte Nebenwirkungen bekannter wie auch neuer Substanzen rascher und zuverlässiger als bisher und mit hoher Nachweisempfindlichkeit festgestellt werden können.

Gegenstand der Erfindung ist demnach ein Verfahren der im Anspruch näher gekennzeichneten Art, bei welchem der Gehalt an Plasmapolypen aus exzidiertem Gewebe in histologischen Schnitten bestimmt wird, und das dadurch gekennzeichent ist, daß man die Bestimmung der Plasmapolypen an der gesamten Magenschleimhaut vornimmt.

Die meisten Medikamente, Substanzen des täglichen Bedarfs und der Nahrung gelangen zunächst in den Magen mit unterschiedlicher Verweildauer. Sind im applizierten Material Schadstoffe vorhanden, läßt sich die Applikationsdauer und Applikationsform definiert und reporduzierbar darstellen.

Grundlage für die vorliegende Erfindung ist die Erkenntnis, daß die Bildung von Plasmapolypen ein allgemein brauchbar und gültiger Indikator für eine bestimmte Art von zellschädigender Wirkung in Abhänigkeit von der Testsubstanz ist.

Das erfindungsgemäße Testverfahren kann vielfältig angewandt werden, um festzustellen, ob die getestete Substanz schädlich auf menschliche und tierische Organe einwirkt. Als geschlechtsunabhängiges Gewebe wird die Magenschleimhaut für die Untersuchung eingesetzt und an ihr die dosisbezogene Schadwirkung getestet.

Das erfindungsgemäße Verfahren mit der Bestimmung der Plasmapolypen an der Magenschleimhaut kann in jedem gewerblichen Betrieb und behördlichen Überwachungslaboratorium vorgenommen werden.

Im Gegensatz zu den üblichen Bestimmungsmethoden für Plasmapolypen, beispielsweise an der Placenta, gelangen die auf der großen Oberfläche der Magenschleimhaut gebildeten abgeschnürten Plasmapolypen fremdpartikelfrei (von Blut und sonstigen Gewebepartikeln) direkt in das Magenlumen und können dort durch Herausnahme und Aufarbeitung des gesamten Magengewebes mit genormter Schnittführung an der Cardia und am Pylorus gewonnen werden. Eine solche fremdpartikelfreie Gewinnung der Plasmapolypen (PP) ist überraschend und aus dem Stand der Technik nicht abzuleiten. Darüberhinaus bietet das Verfahren einen großen methodischen Fortschritt sowohl in der Handhabung als auch in der Möglichkeit der Früherkennung von Schadwirkungen.

Durch das erfindungsgemäße Verfahren wird im Gegensatz zu den üblichen Testverfahren mit geringem Aufwand ein quantitatives Maß für eine zellschädigende Wirkung gewonnen. Hinzu kommt, daß auf diese Weise schleichende zellschädigende Wirkungen einer Substanz, die bisher nur nach langer Einwirkungs- oder Verzögerungszeit histologisch qualitativ gesichert werden konnten nunmehr zuverlässig quantitativ und schnell bestimmbar sind.

Die quantitative Erfassung unter Auszählung der Größenverteilung ist bei Versuchen an der Magenschleimhaut einfach und übersichtlich zu erreichen. Darüberhinaus bietet das erfindungsgemäße Verfahren die Möglichkeit, die PP-Zahlen in Abhängigkeit von Dosis und Einwirkzeit zu studieren.

So ist es möglich, beispielsweise die Schädigungen durch Einwirkung von Acetylsalicylsäure zeitabhängig auch in den tieferen Zellschichten in einfachen histologischen Schnitten zu erfassen. Dadurch wird die Möglichkeit geschaffen, bei wiederholter Applikation oder bei erhöhter Einzeldosis alle Stadien einer Schädigung von einfachen Läsionen der Magenschleimhaut über Ulcerationen bis hin zur Blutung zu beobachten, da wiederholte Applikation oder Überdosierung auch mit einer Schädigung des Gefäßendothels einhergeht. Unter Vorschaltung der PP-Auswertung ist man in der Lage, eventuelle Zellschädigungen oder unerwünschte Nebenwirkungen durch jedweden Stoff dosisabhängig und zeitlich abgestuft zu kontrollieren, wie es bisher nicht möglich war.

Die Auszählung und Bestimmung der Größenverteilung wird mikroskopisch vorgenommen, wobei Besonderheiten nach Form und Inhalt fotografisch festgehalten werden können.

Die PP-Bildung an der Magenschleimhaut erfolgt an Einzelzellen mit einer relativ einheitlichen Größenverteilung von 1,0 bis 3,0 µm Durchmesser. Quantitative Aussagen über die Schadwirkung einer Substanz sind nach dem erfindungsgemäßen Verfahren an einem Tag zu erhalten.

Bei dem erfindungsgemäßen Verfahren werden als Versuchstiere Meerschweinchen, Hamster, Ratten, Mäuse, Hunde, Katzen, Schweine und Affen eingesetzt, wobei die zu testende Schadsubstanz mit Schadwirkung z.B.Acetylsalicylsäure, Pyridoxalphosphat, Pyridoxal, Formaldehyd, Monojodacetat oder das zu testende Gemisch aus Schadsubstanz und Natriumpyruvat intravenös, intraperitoneal oder intramuskulär oder peroral appliziert wird. Die genannten Tierarten reagieren hinsichtlich der zellschädigenden Wirkung ähnlich dem Menschen.

Bezüglich näherer erfindungsgemäßer Einzelheiten wird auf den anschließenden Versuchsteil verwiesen.

Bei positivem Test ist der Beweis erbracht, daß eine schädigende Substanz mit vermehrter PP-Bildung auf der Magenschleimhaut vorliegt. Hier ist z.B. zu erwägen, ob die schädigende Substanz aus dem Verkehr zu ziehen ist oder aber mit einer die zellschädigende Wirkung aufhebenden weiteren Substanz kombiniert werden soll. Als Beispiele für solche Schutzsubstanzen seien genannt: Na-Pyruvat, Na-Lactat, Methylglyoxal, Alanin, Serin, Glutaminsäure, Glutamin, Asparaginsäure, Asparagin, 2-Oxo-dicarbonsäuren und 2-Oxo-tricarbonsäuren.

Die PP-Bildung in der Magenschleimhaut der Ratte erfolgt primär an den oberflächlichen Zellschichten der Magenschleimhaut.

Die PP werden unmittelbar in das Magenlumen abgestoßen. Der Durchmesser der Plasmapolypen liegt bei 1 bis 5 µm, insbesondere 1 bis 2 µm. Plasmapolypen mit einem Durchmesser von 3 bis 5 µm sind die Ausnahme und machen nur 3 bis 5 % der Gesamtzahl aus.

Nach Belastung mit Testsubstanzen, wie Acetylsalicylsäure (ASS),läßt sich in Abhängigkeit von der Verweildauer der Substanz im Magen verfolgen, wann tiefere Schichten der Magenschleimhaut erfaßt werden und wie sich die Testsubstanz auf das Gefäßendothel auswirkt, d.h. ob es gegebenenfalls zu Blutungen kommt. Das austretende Blut gelangt mit den PP ins Magenlumen.

Beispiele

Die PP-Bildung, deren Konzentration und Auszählung wurden an Gruppen zu je 5 Ratten von 140 bis 160 g Körpergewicht, im Mittel 150 g, kontrolliert und die Anzahl der PP auf 1 g Magen (Leergewicht) umgerechnet. Die Tiere waren vor dem Versuch 15 bis 17 Stunden nahrungsfrei. Die Applikation der Testsubstanzen, die gelöst sind in Krebs-Ringer-Lösung (KRL) oder Aq.bidest, erfolgte per Schlundsonde. Die verabreichte Lösung besitzt einen pH-Wert von 7,0 bis 7,2. Die Verweildauer des applizierten Volumens

0147498

hängt zum Teil ab von den Eigenschaften der Testsubstanz. Nach Aq.bidest, $NaHCO_3$, Na-Lactat, aber auch nach Nicotinsäureamid ist sie wesentlich kürzer als nach ASS, Pyridoxol etc. Die Verweildauer der Testsubstanz bis zur Entnahme der Mägen einer Gruppe betrug etwa 20 bis maximal 30 min. Dabei werden dosisabhängig die abgeschnürten PP erfaßt. Die Zahlen sind nicht identisch mit der maximal möglichen PP-Bildung der Magenschleimhaut.

Durch die Versuche wurde festgestellt, daß unter gleichzeitiger Gabe von Na-Pyruvat die Magenschleimhaut geschützt wird, wie sich aus den verminderten PP-Zahlen ablesen läßt.

Das Auftreten von Läsionen, Ulcerationen und Blutungen wird z.B. schon weitgehend unterdrückt, wenn man pro Millimol ASS gleichzeitig 2 Millimole Na-Pyruvat verabreicht.

Es wurden 5 Tierversuche - zu einer Serie zusammengefaßt - durchgeführt innerhalb von 40 min, wobei die Mägen wie folgt aufgearbeitet wurden:

1. Jeder Ratte werden in nicht narkotisiertem Zustand 4,5 ml der Lösung bestehend aus der in KRL oder Aq.bidest gelösten Testsubstanz mittels einer Schlundsonde appliziert.

2. Nach einer Einwirkzeit von etwa 15 bis 20 min werden 40 bis 45 mg Nembutal intraperitoneal appliziert, 2 bis 3 min zugewartet und dann der Magen während eines Zeitraums von etwa 5 min entnommen.

3. Der Magen wird aufgeschnitten, der Inhalt aufgefangen, auf PP geprüft und der leere Magen in. oxygenierte KRL-Lösung mit Glucosezusatz geworfen.

0147498

4. Alle 5 Mägen werden in der glucosehaltigen KRL dreimal hochtourig homogenisiert, das Homogenisat über ein feinmaschiges Sieb gegossen, und das Sieb wird mit 20 ml KRL nachgespült, so daß ein Endvolumen von 80 ml vorliegt.

5. Die Volumina aus den Stufen 3 und 4 werden mit 8 ml eine: 7 %iger Lösung aus einem Copolymeren der Saccharose mit Epichlorhydrin unterschichtet und bei 2000 Umdrehungen/min im Schwingbecherrotor für 20 min zentrifugiert.

6. Die die PP enthaltende Schicht wird erneut 60 min im Festwinkelrotor bei 13000 Umdrehungen/min (Ultrazentrifuge) zentrifugiert, der Überstand wird verworfen und das Sediment weiterverarbeitet.

7 Das Sediment wird einer umgekehrten Zentrifugation in der Ultrazentrifuge bei 25000 U/min für 45 min unterzogen.

8. Die in einer Bohrung aufgefangenen PP aus der umgekehrten Zentrifugation werden in 50 µl KRL aufgenommen.

9. Zur Auszählung der PP aus Stufe 8 bei einem Ansatz von jeweils 3 µl dient ein Phasenkontrastmikroskop. Es werden 10 Felder mit dem Rasterobjektiv ausgezählt, zum Teil unter fotografischer Kontrolle. Die in der nachfolgenden Tabelle aufgeführten Werte stammen jeweils aus drei Ansätzen einer Probe von 5 Rattenmägen oder aus der Auswertung von 30 Feldern.

10. Mittelwert pro Feld x Umrechnungsfaktor pro Feld x 16,6

6

ergibt die Zahl der PP pro Gramm Magen. Der Umrechnungsfaktor für die Feldfläche von 18 x 18 mm² ist 24,652.

Es wurden die in der folgenden Tabelle angegebenen Substanzen injiziert und dann die Anzahl der PP aus einer Serie
von 5 Tieren als Mittelwert pro Gramm Rattenmagen oder gemittelt aus mehreren Serien mit jeweils 5 Tieren ermittelt.

## Tabelle

| Nr. | Substanz | Anzahl der PP pro 1 g Rattenmagen | Anzahl der Serien (5 Ratten = 1 Serie) |
|---|---|---|---|
| 1. | 4,5 ml Aq.bidest | etwa 238.000 | 6 |
| 2. | 4,5 ml Aq.bidest mit 1,33 mmol $NaHCO_3$ | 333.700 | 4 |
| 3. | 4,5 ml Aq.bidest mit 2,0 mmol Na-Lactat | 344.000 | 4 |
| 4. | 4,5 ml Aq.bidest mit 0,5 mmol Na-Pyruvat | etwa 220.000 | 1 |
| 5. | 4,5 ml Aq.bidest mit 0,8 mmol Pyridoxol | 583.000 | 1 |
| 6. | 4,5 ml Aq.bidest mit 2,1 mmol Pyridoxol | 1.039.000 | 1 |
| 7. | 4,5 ml Aq.bidest mit 0,25 mmol ASS* | 610.700 | 3 |
| 8. | 4,5 ml Aq.bidest mit 0,5 mmol ASS* | 728.000 | 3 |
| 9. | 4,5 ml Aq.bidest mit 0,25 mmol ASS* und 0,5 mmol Na-Pyruvat | 358.000 | 2 |
| 10. | 4.5 ml Aq.bidest mit 0,5 mmol ASS* und 1,0 mmol Na-Pyruvat | 460.000 | 2 |

*ASS = Acetylsalicylsäure

- /J -

0147498

Die PP-Bildung setzt nach kurzer, von der Struktur der jeweiliggen Testsubstanz abhängigen Latenzzeit ein und läuft überwiegend während der ersten 30 min ab. In den anschließenden 90 min verringert sich die PP-Bildung ständig und erlischt im allgemeinen nach 2 h. Die Einwirkzeit der Testsubstanz liegt bei etwa 3 min bis 90 min, wobei der Zeitraum der maximalen PP-Bildung von der jeweiligen Testsubstanz abhängt. Die Versuchsergebnisse der Tabelle zeigen, daß bei Verabreichung von Na-Pyruvat keine Erhöhung der PP-Zahlen gegenüber der Blindprobe (4,5 ml Aq.bidest) eintritt. Bei gleichzeitiger Verabreichung von Na-Pyruvat und ASS wird jedoch eine erhebliche Reduzierung der durch die Schadsubstanz ASS induzierten Plasmapolypenbildung erreicht.

1. Prof. Dr. med. Werner Thorn          Z 2155-EU DrK/we
   Flemingstrasse 11
   2000 Hamburg 60

2. Institut Dr. Ziegler
   Wyhlenweg 18
   CH-4126 Bettingen/Basel

Verfahren zum Testen von chemischen
und biologischen Substanzen

Patentansprüche

1. Verfahren zum Testen von chemischen und biologischen Substanzen, die für Arzneimittel, Lebensmittel, Insektizide, Pestizide, Lösungsmittel und andere Güter des täglichen Bedarfs vorgesehen oder in ihnen enthalten sind im Hinblick auf ihre zellschädigende Wirkung, wobei tierisches oder menschliches Gewebe mit den Substanzen, gegebenenfalls zusammen mit Natriumpyruvat, unter Bildung von Plasmapolypen (zellorganellarme Protrusionen), welche als Maß für die zellschädigende Wirkung zu gelten haben, behandelt wurde und der Gehalt an Plasmapolypen nach der Einwirkung der Substanzen in dem behandelten, exzidierten Gewebe bestimmt wird,
d a d u r c h   g e k e n n z e i c h n e t ,
daß man die Bestimmung der Plasmapolypen (PP) in dem exzidierten Gewebe durch Stichproben in histologischen Schnitten qualitativ und an der gesamten Magenschleimhaut quantitativ vornimmt.

2. Verfahren nach Anspruch 1, dadurch gekennzeichnet, daß man die Plasmapolypen auszählt, die nach der Einwirkzeit in das Magenvolumen abgestoßen wurden und die sich aus der Magenschleimhaut gebildet haben, wobei man die Auszählung nach der Zerkleinerung des Magens in flüssiger Phase vornimmt.

3. Verfahren nach Anspruch 2, dadurch gekennzeichnet, daß die Einwirkzeit der zu untersuchenden Substanz auf die Magenschleimhaut 3 bis 90 min beträgt.

4. Verfahren nach Anspruch 3, dadurch gekennzeichnet, daß die Einwirkzeit 15 bis 30 min beträgt.

5. Verfahren nach Anspruch 1, dadurch gekennzeichenet, daß man als zellschädigende Substanz Acetylsalicylsäure und als Schutzsubstanz Natriumpyruvat im Molverhältnis 1 zu 2 in den Magen einbringt.